# EUROPEAN PATENT APPLICATION

(11) **EP 4 205 727 A1**
(43) Date of publication of application: **05.07.2023**
(21) Application number: 22159384.1
(22) Date of filing: 01.03.2022
(51) Int. Cl.: A61K 8/49, A61K 8/24, A61K 8/35, A61K 8/368, A61K 8/60, A61Q 15/00

(54) **A LOW-SKIN IRRITATING DEODORANT COMPOSITION AND A METHOD FOR PREPARING IT**

(30) Priority: 31.12.2021 CN 202111668847; 28.02.2022 HK 32022049017
(71) Applicant: Sislan (Zhuhai) Pharmaceutical Technology Co., Ltd, Zhuhai, Guangdong (CN)
(72) Inventor: LAU, Lo Lulu, Hong Kong (HK); CHENG, Hung, Hong Kong (HK); WANG, Zhentao, Nanyang (CN); PAN, Jieying, Nanyang (CN); LI, Xiaotian, Zhengzhou (CN); WANG, Zhehuan, Zhengzhou (CN); GUO, Hui, Zhengzhou (CN)
(74) Representative: Bewley, Ewan Stuart

(57) **Abstract**

The present invention relates to a low-skin irritating deodorant composition. The composition comprises an effective amount of an active ingredient comprising hexamine, derivatives thereof, or a combination thereof for reducing, inhibiting or suppressing development of malodour from a body part; a buffer system for substantially neutralising topical acidity of the body part; and an anti-irritating agent in an effective amount, wherein the anti-irritating agent comprises one or more botanically derived compounds selected from a group consisting of flavonoids, alkaloids, iridoids, terpenes, phenols, or a combination thereof.

## Description

### Field of the Invention

The invention relates to a composition for use in a deodorant for a body part of a subject, and particularly, but not exclusively, to a low-skin irritating deodorant composition.

### Background of the Invention

Body odor generally refers to a condition in which an unpleasant smell or a malodor is released from a body part or skin of a body part of a human subject. Although body odor is known to be influenced by various physiological conditions, it is generally related to perspiration or sweating of the subject, and the subsequent bacterial decomposition of the organic compounds present in the sweat which forms the malodor carrying volatile organic acids. It is therefore most common for body odor to develop at axillary regions of the body such as the underarm areas where a large number of sweat glands are located, although body odor may also develop at other parts of the human body such as the palms of the hands, the soles of the feet, the upper thighs and the back region.

Various deodorant compositions and/or preparations have been developed to reduce, alleviate, eliminate or mask the unpleasant smell, and/or to suppress or inhibit the production of sweat from the skin of the body parts. In general, the deodorants may work by providing an antiseptic effect to inhibit the bacterial decomposition; by incorporating an anti-perspiring agent to suppress or inhibit perspiration; and/or by introducing fragrance to mask the generated odor. Deodorant preparations may come in different forms, such as solid, gel, cream, liquid or aerosol for various applications such as apply-on or roll-on sticks or rollers, body sprays or even body wipes; and can be classified as a cosmetic or a pharmaceutical product depending on the specific active ingredients in the composition. A number of commonly used deodorizing actives include alcohols, triclosan, sodium stearate, ethylenediaminetetraacetic acid (EDTA), chlorhexidine and formaldehyde, etc. for their antibacterial function; and/or salts of aluminum or zinc such as aluminum chloride, aluminum chlorohydrate, and aluminum-zirconium compound, etc. as antiperspirants. In addition to these commonly used actives, hexamine, which is also known as hexamethylenetetramine or methenamine, is found to be a potent deodorizing agent, although its deodorizing mechanism is yet to be clearly understood. Nevertheless, hexamine comprising deodorant preparations have been reported to cause allergic reaction or skin irritation to the users, which may potentially relate to the release of formaldehyde from hexamine in use. For example, Susak Z, Minkov R, Isakov E. The use of Methenamine as an antiperspirant for amputees. Prosthet Orthot Int. 1996 Dec; 20(3):172-5*,* Bergstresser PR, Quero R. Treatment of hyperhidrosis with topical methenamine. Int J Dermatol. 1976 Jul-Aug; 15(6):452-5*,* and Cullen SI. Topical methenamine therapy for hyperhidrosis. Arch Dermatol. 1975 Sep;111(9):1158-60 discussed the use of hexamine as topical active and the problem of contact allergic reaction associated with the use of hexamine in topical formulation since early 1970s. Yet very few solutions have been identified other than limiting the use amount of hexamine to a low dose of about 5% or below. In this regard, the Applicant's team has previously reported in US patent no. US 10,596,092 B2 a topical composition comprising 20-40% of hexamine having a buffer system for stabilizing hexamine to reduce or slow down formation of formaldehyde in the composition to thereby minimise the skin-irritation caused by formaldehyde. Nevertheless, it remains a challenge to apply hexamine in topical formulations, and the undesirable skin irritation caused by hexamine and the degradation product is yet to be solved.

### Objects of the Invention

An object of the present invention is to provide a low-skin irritating deodorant composition.

Another object of the present invention is to provide a low-skin irritating composition for use in deodorant preparations.

A further object of the present invention is to mitigate or obviate to some degree one or more problems associated with known deodorant compositions and/or preparations, or at least to provide a useful alternative.

The above objects are met by the combination of features of the main claims; the subclaims disclose further advantageous embodiments of the invention.

One skilled in the art will derive from the following description other objects of the invention. Therefore, the foregoing statements of object are not exhaustive and serve merely to illustrate some of the many objects of the present invention.

### Summary of the Invention

In general, the invention provides a low-skin irritating composition for use in topical application such as, but is not limited to, a deodorant composition for reducing, inhibiting or suppressing malodor developed from a body part of a subject such as a human subject. Particularly, the invention provides a hexamine-comprising composition for use in a deodorant preparation which demonstrates low, or a significantly reduced skin irritation to the subject even when the deodoring active is provided at sufficiently high dose to effectively reduce, inhibit or suppress development of sweat relating malodor.

In a first main aspect, the invention provides a low-skin irritating deodorant composition. The composition comprises an effective amount of an active ingredient comprising hexamine, derivatives thereof, or a combination thereof for reducing, inhibiting or suppressing development of malodour from a body part; a buffer system for substantially neutralising topical acidity of the body part; and an anti-irritating agent in an effective amount, wherein the anti-irritating agent comprises one or more botanically derived compounds selected from a group consisting of flavonoids, alkaloids, iridoids, terpenes, phenols, or a combination thereof.

In a second main aspect, the invention provides a deodorant preparation for use at a body part of a subject. The deodorant preparation comprises the composition according to the first main aspect.

In a third main aspect, the invention provides a method of preparing a low skin-irritating deodorant composition. The method comprises the steps of providing a buffer solution; dissolving an effective amount of hexamine, derivatives thereof, or a combination thereof in the buffer solution to form a hexamine-comprising solution; and incorporating an anti-irritating agent in an effective amount into the hexamine-comprising solution, wherein the anti-irritating agent comprises one or more botanically derived compounds selected from a group consisting of flavonoids, alkaloids, iridoids, terpenes, phenols, or a combination thereof.

The summary of the invention does not necessarily disclose all the features essential for defining the invention; the invention may reside in a sub-combination of the disclosed features.

### Brief Description of the Drawings

The foregoing and further features of the present invention will be apparent from the following description of preferred embodiments which are provided by way of example only in connection with the accompanying figure, of which:
Fig. 1 shows the skin condition of a tested guinea pig having moderate irritation;
Fig. 2 shows the skin condition of a tested guinea pig having slight irritation;
Fig. 3 shows the skin condition of a tested guinea pig having atopic dermatitis;
Fig. 4A shows the skin condition of a tested guinea pig having atopic dermatitis before treatment by saline solution as a control experiment;
Fig. 4B shows the skin condition of a tested guinea pig having atopic dermatitis after treatment by saline solution as a control experiment;
Fig. 5A shows the skin condition of a tested guinea pig having atopic dermatitis before treatment by a deodorant composition having no anti-irritating agent;
Fig. 5B shows the skin condition of a tested guinea pig having atopic dermatitis after treatment by a deodorant composition having no anti-irritating agent;
Fig. 6A shows the skin condition of a tested guinea pig having atopic dermatitis before treatment by a deodorant composition having piperine as an anti-irritating agent;
Fig. 6B shows the skin condition of a tested guinea pig having atopic dermatitis after treatment by a deodorant composition having piperine as an anti-irritating agent;
Fig. 7A shows the skin condition of a tested guinea pig having atopic dermatitis before treatment by a deodorant composition having a combination of piperine and matrine as an anti-irritating agent; and
Fig. 7B shows the skin condition of a tested guinea pig having atopic dermatitis after treatment by a deodorant composition having a combination of piperine and matrine as an anti-irritating agent.

### Description of Preferred Embodiments

The following description is of preferred embodiments by way of example only and without limitation to the combination of features necessary for carrying the invention into effect.

Reference in this specification to "one embodiment" or "an embodiment" means that a particular feature, agent, composition, preparation or characteristic described in connection with the embodiment is included in at least one embodiment of the invention. The appearances of the phrase "in one embodiment" in various places in the specification are not necessarily all referring to the same embodiment, nor are separate or alternative embodiments mutually exclusive of other embodiments. Moreover, various features are described which may be exhibited by some embodiments and not by others. Similarly, various requirements are described which may be requirements for some embodiments but not other embodiments.

The present invention relates to a composition for use in topical application such as, but is not limited to, a deodorant composition for reducing, inhibiting or suppressing malodor developed from a body part of a subject such as a human subject. Particularly, the invention provides a hexamine-comprising composition for use in a deodorant preparation which demonstrates low, or a significantly reduced skin irritation to the subject even when the deodoring active is provided at sufficiently high dose to effectively reduce, inhibit or suppress development of sweat relating malodor. The composition is generally applicable for use on any body parts such as axillary regions of the human body including underarm areas, where a large number of sweat glands are present. Nevertheless, a person skilled in the art would understand that the present invention shall not be limited to axilla applications, but also applicable to any other suitable body parts where sweating is likely to occur, which may include, but is not limited to, hands, feet, upper thighs and/or back region.

In the context of the present invention, the term "deodorant" relates in broad terms to encompass any agents, compositions, preparations, formulations and/or products adapted to reduce, inhibit, prevent, suppress, eliminate, mask odor, and more specifically, malodor; and that the deodorizing actions can be achieved by any known and possible mechanisms, such as but not limited to, the incorporation of antiperspirant, antibacterial agent, disinfectant or fragrance into the composition. The term "deodorant" in the context of the present invention therefore encompasses compositions and products in both cosmetic and pharmaceutical applications.

The terms "skin irritating" and "skin irritation" as used in the context of the present invention generally relate to the development of any sensitive, allergic or inflammatory reactions to skin such as rash or dermatitis. Skin irritation may cause itchy, red, dry, or swollen skin, and may lead to blisters, patches or crusts of skin. The term "anti-irritating agent" or "anti-irritant" therefore has been used to generally describe any agent, compound or group of agents or compounds capable of reducing potential irritation caused by other ingredients in a composition or a formulation. The anti-irritating agent can generally be included to a formulation to benefit tolerability of the product, to counter any potential allergic reaction of the active, and to allow a soothing or calming effect to the product.

Hexamine, which is also known as hexamethylenetetramine or methenamine, has been traditionally used as a pharmaceutical active ingredient for controlling urinary tract infection by releasing formaldehyde and ammonia in acidic urine, with formaldehyde being potent to stop growth of bacteria in the urinary track. Recently, increasing attention has been made to the use of hexamine as an active ingredient in deodorant formulations, although the relevant deodorizing mechanism is not yet clearly understood. One widely acceptable presumption is that hexamine, in the presence of sweat with a natural pH of about 4.0 to 7.0, releases formaldehyde which possesses antibacterial property. Yet it was revealed from previous study of the Applicant's team that hexamine, when prepared in basic condition, is still capable of demonstrating an antibacterial effect. In addition, hexamine itself is a weak base and is found in the study as being able to neutralize a number of odoriferous, volatile organic acids commonly formed from the bacterial decomposition of organic matter in the sweat. It was further demonstrated by the study of the Applicant's team that the hexamine comprising composition is adapted to inhibit aminoacylase enzymatic reaction, which is reported to be the mechanism by which organic matter in sweat is decomposed by bacteria which causes body odor. Lastly, formaldehyde released from hexamine is known to form keratin plugs at distal sweat ducts, which substantially block the opening of hair follicles and thus result in a significant reduction or suppression in sweat release. Accordingly, hexamine is found to play a multifunctional role in reducing or inhibiting the development of malodor in a deodorant composition and/or preparation.

Nevertheless, hexamine comprising deodorant preparations have also been reported to cause skin irritation. One possible cause of the skin irritation may relate to the release of formaldehyde, which is considered as a potential skin irritant and particularly, when the formaldehyde is present in high concentration such as in a highly acidic environment, in which the breakdown of hexamine to form formaldehyde as a by-product would be aggravated.

In the context of the present invention, the term "by-product" as used in relation to formaldehyde, or any aldehydes in general, does not preclude their possessing and/or exerting any functional characteristics. For example, the released formaldehyde from the decomposition of hexamine or hexane-comprising ingredient may serve as a subsidiary active to provide an additional or to support the deodorizing effect along with the hexamine or hexamine-comprising active ingredient, depending on the specific composition, preparation and application.

Accordingly, one embodiment of the present invention relates to a low or reduced skin-irritating or non-skin irritating deodorizing composition for, for example, axilla application of a human subject. The composition comprises an active ingredient in an effective amount sufficient for reducing, inhibiting or suppressing any developed malodor caused by sweat at the body part. The active ingredient may comprise one or more of a hexamine, a derivative thereof, and/or a combination thereof. In one embodiment, the active ingredient is of about 1.0 w/w% to about 42.0 w/w% in respect of a total weight of the composition.

The composition further comprises a buffer system for substantially neutralising topical acidity of the body part to stabilize the acid-sensitive active ingredient, and thus, controlling or preventing release of any aldehydes such as formaldehyde as a result of hexamine decomposition. The buffer system is adapted to maintain pH of the composition within a range of about 7.0 to about 11.0, and preferably, about 7.5 to about 10.0 after the composition is applied on the body part. The buffer system also provides a slightly basic to basic environment for neutralization of odoriferous organic acids resulting from the bacterial decomposition of organic compounds present in the sweat, which further assists in reducing any developed malodor.

In one embodiment, the buffer system may comprise one or more commonly known pH buffers adapted to provide the required buffer range as above described, such that the hexamine or hexamine comprising active ingredient in the composition can be substantially stabilized and that the effect of the acidic sweat can be substantially minimized. The buffer system may comprise one or more of an inorganic compound and an organic compound, such as but not limited to, one or more of 3-Morpholinopropane-1-sulfonic acid (MOPS), 4-(2-hydroxyethyl)-1-piperazineethanesulfonic acid) (HEPES), 3-(cyclohexylamino)-1-propanesulfonic acid (CAPS), 3-[[1,3 -dihydroxy-2-(hydroxymethyl)propan-2-yl]amino]-2-hydroxypropane-1-sulfonic acid (TAPSO), 2-Amino-2-(hydroxymethyl)-1,3-propanediol (Trizma) and a phosphate compound, as well as the salts thereof, or a combination thereof. In one embodiment, the phosphate compound of the buffer system comprises one or more of a disodium phosphate and a monosodium phosphate. For example, the buffer system may comprise a phosphate buffer saline (PBS). Preferably, the buffer system may comprise an aqueous buffer solution to facilitate an aqueous formulation of the composition.

In one embodiment, the composition further comprises an anti-irritating agent or an anti-irritant in an effective amount, wherein the anti-irritating agent comprises one or more botanically derived compounds. The term "botanically derived compound" refers to a substance, a compound or an extract which is obtained or derived from a plant or one or more parts of a plant. Botanically derived compounds may include, but are not limited to, herbs or herbal substances, botanical extracts, and natural medicinal substances or preparations such as the traditional Chinese medicines (TCM). In one embodiment, the botanically derived compound of the anti-irritating agent of the present invention is selected from a group consisting of flavonoids, alkaloids, iridoids, terpenes, phenols, or a combination thereof.

Preferably, the botanically derived compounds of the anti-irritating agent comprise flavonoids, wherein the flavonoids are selected from a group consisting of baicalin, quercetin, neohesperidin, hispidulin, or a combination thereof.

Preferably, the botanically derived compounds of the anti-irritating agent comprise alkaloids, wherein the alkaloids are selected from a group consisting of piperine, matrine, or a combination thereof.

Preferably, the botanically derived compounds of the anti-irritating agent comprise iridoids, wherein the iridoids comprise iridoid glycosides; and more preferably, the iridoid glycosides may comprise geniposide.

Preferably, the botanically derived compounds of the anti-irritating agent comprise terpenes, wherein the terpenes are selected from a group consisting of monoterpenes, diterpenes, triterpenes, sesquiterpenes, sesquarterpenes, tetraterpenes, or a combination thereof. More preferably, the botanically derived compounds of the anti-irritating agent comprise monoterpenes, wherein the monoterpenes may comprise total paeony glucosides (TPG) which may comprise one or more substances such as paeoniflorin, albiflorin, oxypaeoniflorin, and benzoylpaeoniflorin. More preferably, the botanically derived compounds of the anti-irritating agent comprise triterpenes, wherein the triterpenes may comprise celastrol.

Preferably, the botanically derived compounds of the anti-irritating agent comprise phenols, wherein the phenols can be in the form of or comprise polyphenols. More preferably, the polyphenols may comprise curcumin.

Table 1 below shows the chemical structure of a number of botanically derived compounds of the anti-irritating agent embodied in the present invention. A person skilled in the art would appreciate that the present invention should not be limited to the specific compounds discussed in the embodiments. Instead, any other compounds which fall into the chemical groups of flavonoids, alkaloids, iridoids, terpenes, phenols, or share similar chemical structures to the embodied compounds and thus are reasonably expected to demonstrate similar chemical properties, shall also be encompassed by the present invention.
Table 1. Examples of the botanically derived compounds of the anti-irritating agents embodied in the present invention and their chemical structures.

### Flavonoids

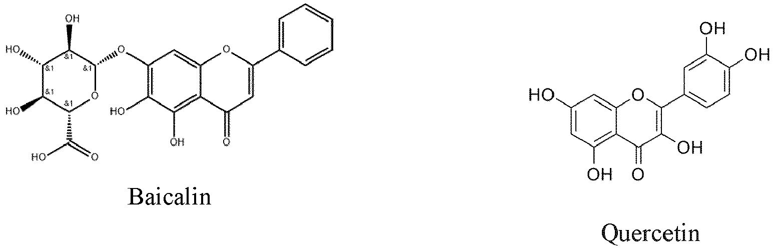

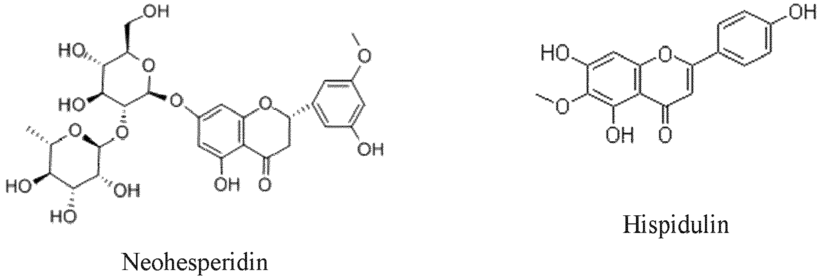

### Alkaloids

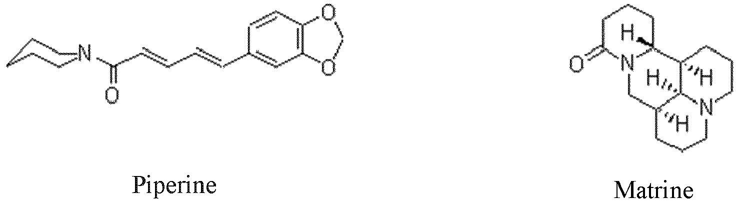

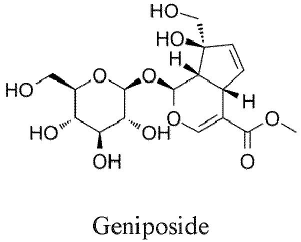

### Terpenes

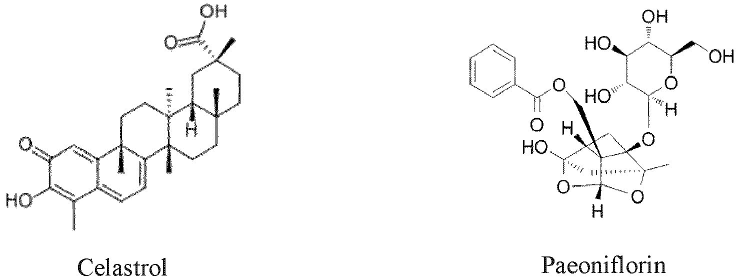

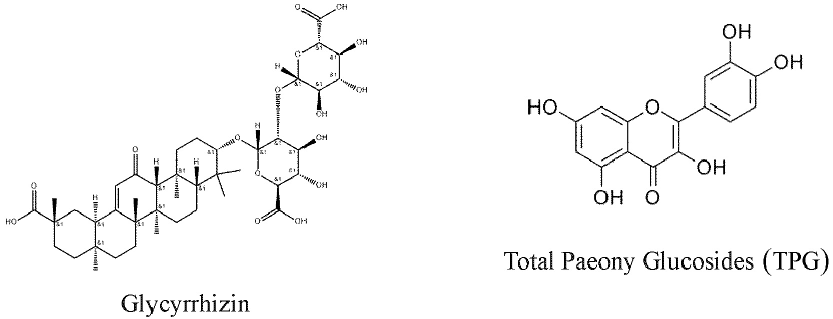

### Phenols

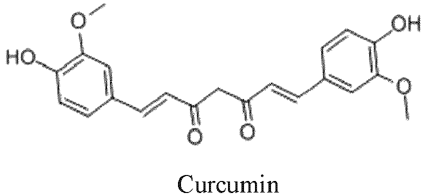

In one embodiment, the anti-irritating agent is of about 0.1 w/w% to about 1.0 w/w% in respect of a total weight of the composition, and more preferably, of about 0.2 w/w% to about 0.4 w/w% in respect of a total weight of the composition.

In one embodiment, the composition may optionally comprise one or more excipients, such as but not limited to, one or more humectants or emollients for topical application such as glycerol; fragrances and essential oils; stabilizers; thickeners; preservatives; antioxidants; cosmetic agents; pH adjusters such as sodium bisulfite, hydrochloric acid and ammonium carbonate; propellants; solvents; cooling agents; colorants; and sun-blocking agents, etc. A person skilled in the art would understand that the composition of the present invention shall not be limited to any specific embodiments or exemplified formulations, but any additions, modifications or variations which are considered suitable or appropriate should also be encompassed.

Optionally, the composition may comprise one or more anti-bacterial agents and/or antifungal agents to further enhance the antiseptic effect, for example, disinfectants such as quaternary ammonium salts, or biocides such as chlorhexidine. The composition may also comprise one or more chelating agents such as ethylenediaminetetraacetic acid (EDTA) for inhibiting the aminoacylase enzymatic reaction of bacteria, which is known to generate odoriferous organic acids causing the malodor.

The present invention also relates to a deodorising preparation or a deodorant for use at a body part of a subject, comprising one or more of the above described embodiments of the composition. The deodorant is preferred to be topically applied or administered, and can be provided in one or more known forms of preparation, such as but not limited to, a solid, a gel, a cream, a powder, a liquid, a spray or a disposable wipe, i.e. a fabric or tissue paper impregnated with the composition in liquid form.

The present invention may further relate to a method of preparing a low or reduced skin-irritating composition for use in reducing, inhibiting or suppressing malodour from a body part of a subject. In one embodiment, the method comprises the step of providing a buffer solution, such as by dissolving an appropriate amount of disodium hydrogen phosphate and sodium dihydrogen phosphate into deionized water to form a phosphate buffer solution; dissolving an effective amount of hexamine, derivatives thereof, or a combination thereof in the buffer solution with mixing to form a hexamine-comprising solution; and incorporating an anti-irritating agent in an effective amount into the hexamine-comprising solution with mixing. The anti-irritating agent comprises one or more botanically derived compounds selected from a group consisting of flavonoids, alkaloids, iridoids, terpenes, phenols, or a combination thereof. For example, the botanically derived compounds may comprise, but are not limited to, one or more of baicalin, celastrol, quercetin, total paeony glucosides (TPG), curcumin, neohesperidin, piperine, paeoniflorin, hispidulin, glycyrrhizin, matrine and/or geniposide.

Preferably, the step may further comprise including a solvent into the hexamine-comprising solution prior to incorporating the anti-irritating agent into the hexamine-comprising solution. The solvent may also be a humectant such as glycerol. The step may further comprise including one or more excipients as describe above into the hexamine-comprising solution after the incorporating of the anti-irritating agent into the hexamine-comprising solution.

### Experimental

A number of embodied formulations and their corresponding compositions are provided in Table 2 below for preparing buffered, hexamine comprising compositions each having an anti-irritating agent in accordance with the present invention. All of the formulations are prepared based on the method steps as described above.

**Table 2. Exemplified formulations of the low-skin irritating deodorant composition in accordance with the present invention. The concentrations are represented in w/w%.**

| Formulation | Buffer solution | | Hexamine | Glycerol | Anti-irritating Agent | Fragrance | Deionized water |
|---|---|---|---|---|---|---|---|
| | Disodium phosphate | Sodium dihydrogen phosphate | | | | | |
| 1 | 0.2% | 2% | 8% | 2% | 0.1% | 0.8% | Fill up to 100% |
| 2 | 0.4% | 4% | 20% | 4% | 0.2% | 0.5% | Fill up to 100% |
| 3 | 1% | 5% | 35% | 7% | 0.3% | 0% | Fill up to 100% |
| 4 | 0.8% | 8% | 30% | 9% | 0.5% | 0.2% | Fill up to 100% |
| 5 | 0.71% | 2.58% | 40% | 10% | 0.2% | 0.5% | Fill up to 100% |
| 6 | 0.71% | 2.58% | 40% | 10% | 0.4% | 0.5% | Fill up to 100% |

### Embodiment 1

Formulation 5 has been adopted to prepare the deodorant composition in accordance with the present invention for the skin sensitization test as described below. In this experiment, the anti-irritating agents being used in the deodorant samples are, separately, baicalin, celastrol, quercetin, total paeony glucosides (TPG), curcumin, neohesperidin, piperine, paeoniflorin, hispidulin, glycyrrhizin, matrine and geniposide in a concentration of about 0.2 w/w% in respect of the total weight of the composition. The skin sensitization experiment was carried out on healthy guinea pigs, and specifically, 390 guinea pigs were randomly divided into 13 groups, with 30 guinea pigs in each group. Twelve (12) deodorant samples prepared under Formulation 5 each comprising one of the anti-irritating agent at 0.2 w/w%, and one (1) deodorant sample prepared without any anti-irritating agent as a control experiment, were respectively set up for comparative experiments.

On Day 0 to Day 2 daily, the 13 deodorant samples were applied separately to the hair-removed area of the respective guinea pigs at a dosage of 0.2 ml/animal. The area applied with the deodorant sample was then covered with two layers of gauze and one layer of oil paper, and subsequently, sealed and fixed with medical tape for 6 hours. After the 6 hours of application, the coverings were removed. No treatment was given for Day 3 to Day 7, and the stimulation was repeated on the Day 8. Skin conditions of the guinea pigs were observed on Day 9, with the observations being recorded in Table 3 below:

**Table 3. Results of skin sensitization test on guinea pigs.**

| Deodorant sample | Anti-irritating agent (A) at 0.2 w/w%: | Number of animal showing irritation | Degree of irritation |
|---|---|---|---|
| A1 | B aicalin | 2 | Slightly irritating |
| A2 | Celastrol | 1 | Slightly irritating |
| A3 | Quercetin | 1 | Slightly irritating |
| A4 | Total paeony of glucosides | 1 | Slightly irritating |
| A5 | Curcumin | 0 | No irritation |
| A6 | Neohesperidin | 1 | Slightly irritating |
| A7 | Piperine | 0 | No irritation |
| A8 | Paeoniflorin | 1 | Slightly irritating |
| A9 | Hispidulin | 1 | Slightly irritating |
| A10 | Glycyrrhizin | 0 | No irritation |
| A11 | Matrine | 0 | No irritation |
| A12 | Geniposide | 0 | No irritation |
| A13 | Nil (Control) | 4 | 2 Moderately irritating |
| | | | 2 Slightly irritating |

In addition, dinitrochlorobenzene (DNCB)-induced atopic dermatitis experiment on guinea pigs was performed with respect to these 13 deodorant examples. Particularly, 390 guinea pigs were randomly divided into 13 groups, with 30 guinea pigs in each group. On Day 0 to Day 2 daily, 1% DNCB was applied to the hair-removed area on the left side at a dosage of 0.2 ml/animal. The applied area was covered with two layers of gauze and one layer of oil paper, and subsequently, sealed and fixed with medical tape for 6 hours. After the 6 hours of application, the coverings were removed to obtain guinea pigs with allergic dermatitis. Severe irritations were observed in all groups after the DNCB stimulation.

After Day 3, each group was respectively treated by applying the above-described 13 deodorant samples daily for 5 days. The treatment results were recorded in Table 4 as follows:

**Table 4. Results of skin sensitization test after DNCB-induced atopic dermatitis on guinea pigs.**

| Deodorant sample | Anti-irritating agent (A) at 0.2 w/w%: | After 5 days of treatment | | Number of days taken for completely curing all animals |
|---|---|---|---|---|
| | | Number of animals showing relief | Number of animals being cured | |
| A1 | Baicalin | 20 | 3 | 11 |
| A2 | Celastrol | 21 | 6 | 10 |
| A3 | Quercetin | 21 | 5 | 9 |
| A4 | Total paeony of glucosides | 19 | 4 | 10 |
| A5 | Curcumin | 23 | 6 | 9 |
| A6 | Neohesperidin | 18 | 5 | 10 |
| A7 | Piperine | 24 | 6 | 8 |
| A8 | Paeoniflorin | 22 | 4 | 10 |
| A9 | Hispidulin | 18 | 5 | 10 |
| A10 | Glycyrrhizin | 24 | 5 | 9 |
| A11 | Matrine | 23 | 6 | 8 |
| A12 | Geniposide | 25 | 5 | 7 |
| A13 | Nil (Control) | 8 | 0 | 14 |

The results demonstrate that the deodorant composition comprising one of the anti-irritating agents as embodied in the present invention has significantly reduced skin-irritation over the control sample in which no anti-irritation agent is present. The deodorant composition further demonstrates a potent allergy-treating effect over DNCB-induced atopic dermatitis.

### Embodiment 2

Formulation 6 has been adopted in the skin sensitization test as described below. In this experiment, the anti-irritating agent comprise a combination of any two of baicalin, celastrol, quercetin, total paeony glucosides (TPG), curcumin, neohesperidin, piperine, paeoniflorin, hispidulin, glycyrrhizin, matrine and geniposide, each at about 0.2 w/w% to make up a total anti-irritating agent concentration of about 0.4 w/w% in each deodorant sample. Eight (8) deodorant samples have been prepared with the respective irritating agents detailed in Table 5 below.

**Table 5. Deodorant samples prepared for skin sensitization test on guinea pigs.**

| Deodorant sample | Irritating agent (B) |
|---|---|
| B1 | 0.2 w/w% of baicalin and 0.2 w/w% of celastrol |
| B2 | 0.2 w/w% of quercetin and 0.2 w/w% of total paeony glucosides (TPG) |
| B3 | 0.2 w/w% of curcumin and 0.2 w/w% of neohesperidin |
| B4 | 0.2 w/w% of piperine and 0.2 w/w% of paeoniflorin |
| B5 | 0.2 w/w% of hispidulin and 0.2 w/w% of glycyrrhizin |
| B6 | 0.2 w/w% of matrine and 0.2 w/w% of geniposide |
| B7 | 0.2 w/w% of piperine and 0.2 w/w% of matrine |
| B8 | 0.2 w/w% of paeoniflorin and 0.2 w/w% of geniposide |

The skin sensitization experiment was carried out on healthy guinea pigs. Specifically, 240 guinea pigs were randomly divided into 8 groups, with 30 guinea pigs in each group. The above mentioned 8 deodorant samples prepared under Formulation 6, each comprising two botanically derived compound at 0.2 w/w% to make up a total anti-irritating agent concentration of 0.4 w/w%, were respectively set up for comparative experiments.

On Day 0 to Day 2 daily, the 8 deodorant samples were applied separately to the hair-removed area of the respective guinea pigs at a dosage of 0.2 ml/animal. The area applied with the deodorant sample was then covered with two layers of gauze and one layer of oil paper, and subsequently, sealed and fixed with medical tape for 6 hours. After the 6 hours of application, the coverings were removed. No treatment was given for Day 3 to Day 7, and the stimulation was repeated on the Day 8. Skin conditions of the guinea pigs were observed on Day 9 with the observation recorded in Table 6 as follow:

**Table 6. Results of skin sensitization test on guinea pigs.**

| Deodorant sample | Irritating agent (B) | Number of animal showing irritation | Degree of irritation |
|---|---|---|---|
| B1 | 0.2 w/w% of baicalin and 0.2 w/w% of celastrol | 1 | Slightly irritating |
| B2 | 0.2 w/w% of quercetin and 0.2 w/w% of total paeony glucosides (TPG) | 1 | Slightly irritating |
| B3 | 0.2 w/w% of curcumin and 0.2 w/w% of neohesperidin | 0 | No irritation |
| B4 | 0.2 w/w% of piperine and 0.2 w/w% of paeoniflorin | 0 | No irritation |
| B5 | 0.2 w/w% of hispidulin and 0.2 w/w% of glycyrrhizin | 0 | No irritation |
| B6 | 0.2 w/w% of matrine and 0.2 w/w% of geniposide | 0 | No irritation |
| B7 | 0.2 w/w% of piperine and 0.2 w/w% of matrine | 0 | No irritation |
| B8 | 0.2 w/w% of paeoniflorin and 0.2 w/w% of geniposide | 0 | No irritation |

In addition, dinitrochlorobenzene (DNCB)-induced atopic dermatitis experiment on guinea pigs were performed with respect to these 8 deodorant examples. Particularly, 240 guinea pigs were randomly divided into 8 groups, with 30 guinea pigs in each group. On Day 0 to Day 2 daily, 1% DNCB was applied to the hair-removed area on the left side at a dosage of 0.2 ml/animal. The applied area was then covered with two layers of gauze and one layer of oil paper, and subsequently, sealed and fixed with medical tape for 6 hours. After the 6 hours of application, the coverings were removed to obtain guinea pigs with allergic dermatitis. Severe irritations were observed in all groups after the DNCB stimulation.

After Day 3, each group was respectively treated by applying the respective deodorant samples daily for 5 days. The treatment results by the 8 deodorant samples were recorded in Table 7 below:

**Table 7. Results of skin sensitization test after DNCB-induced atopic dermatitis on guinea pigs.**

| Deodorant sample | Irritating agent (B) | After 5 days of treatment | | Number of days taken for completely curing all animals |
|---|---|---|---|---|
| | | Number of animals showing relief | Number of animals being cured | |
| B1 | 0.2 w/w% of baicalin and 0.2 w/w% of celastrol | 21 | 4 | 10 |
| B2 | 0.2 w/w% of quercetin and 0.2 w/w% of total paeony glucosides (TPG) | 22 | 3 | 8 |
| B3 | 0.2 w/w% of curcumin and 0.2 w/w% of neohesperidin | 23 | 5 | 9 |
| B4 | 0.2 w/w% of piperine and 0.2 w/w% of paeoniflorin | 24 | 5 | 8 |
| B5 | 0.2 w/w% of hispidulin and 0.2 w/w% of glycyrrhizin | 22 | 5 | 8 |
| B6 | 0.2 w/w% of matrine and 0.2 w/w% of geniposide | 24 | 6 | 7 |
| B7 | 0.2 w/w% of piperine and 0.2 w/w% of matrine | 23 | 6 | 8 |
| B8 | 0.2 w/w% of paeoniflorin and 0.2 w/w% of geniposide | 24 | 5 | 8 |

The deodorant samples comprising a combination of two botanically derived compounds as the anti-irritating agent, as shown in Embodiment 2, have demonstrated an improved anti-irritating effect over the deodorant samples comprising only one botanically derived comound as the anti-irritating agent, as shown in Embodiment 1. The deodorant composition comprising two botanically derived compounds as the anti-irritating agent further demonstrates potent allergy-treating effect over DNCB-induced atopic dermatitis.

Furthermore, combining three or more anti-irritating agents selected from baicalin, celastrol, quercetin, total paeony glucosides (TPG), curcumin, neohesperidin, piperine, paeoniflorin, hispidulin, glycyrrhizin, matrine and geniposide in the deodorant composition of the present invention further demonstrated enhanced anti-irritating and allergy-treating effects in the guinea pig experiments.

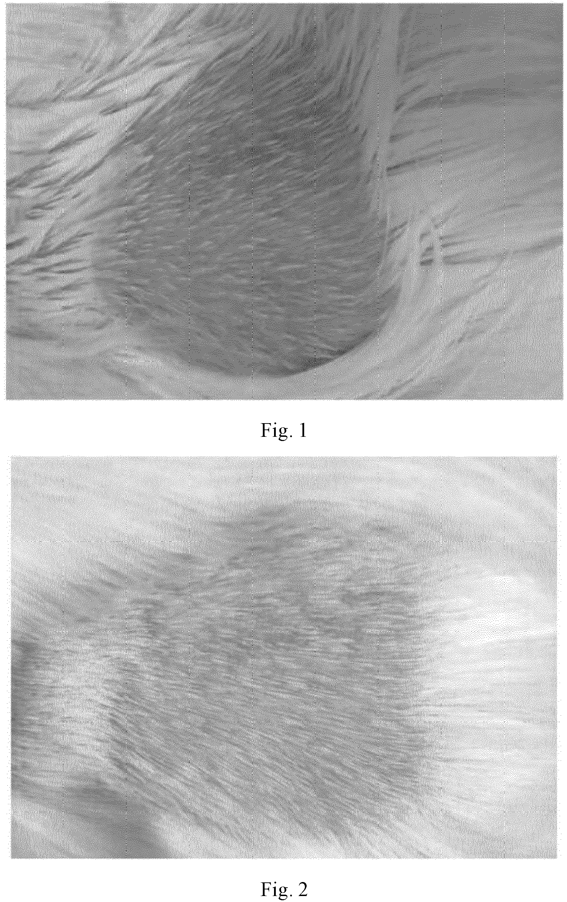

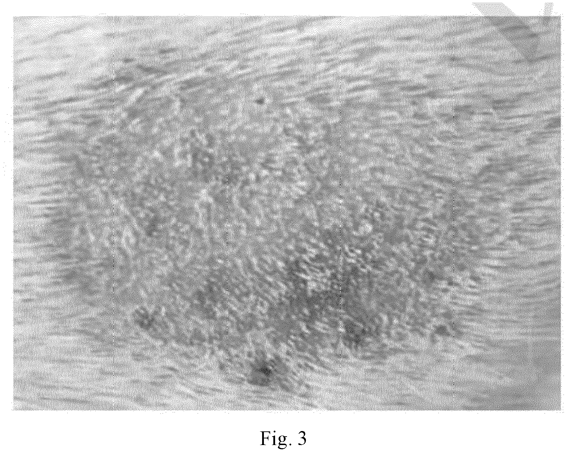

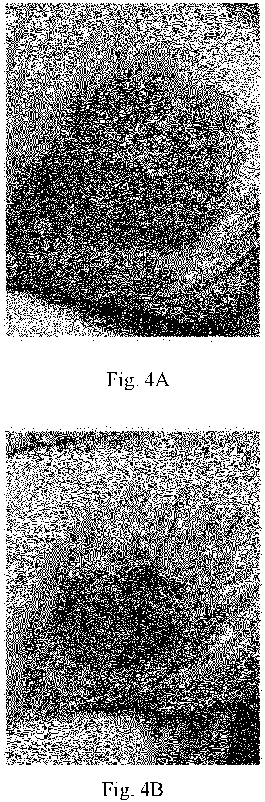

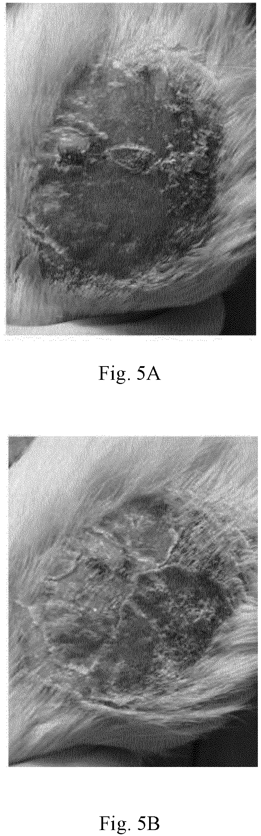

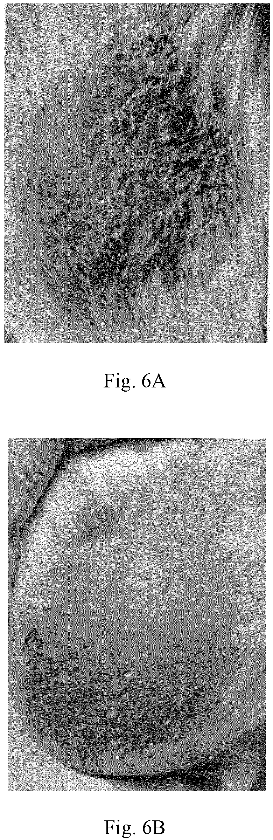

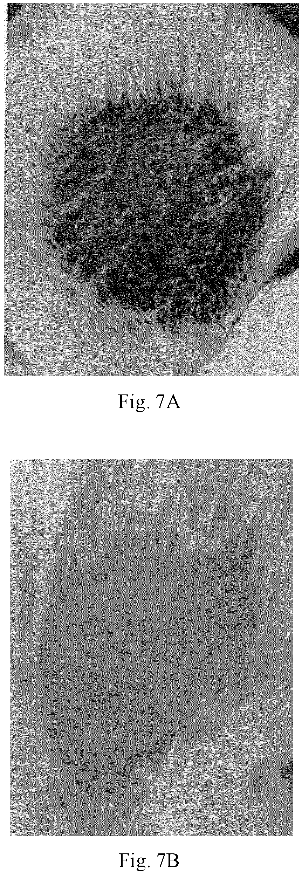

Referring to Figs. 1-3, shown are skin conditions of the tested guinea pigs having moderate irritation, slight irritation and atopic dermatitis, respectively. Figs. 4A and 4B respectively show the skin condition, before and after, the atopic dermatitis being treated by saline solution comprising sodium chloride as control experiments. Figs 5A, 5B respectively show the skin condition, before and after, the atopic dermatitis being treated by deodorant composition having no anti-irritating agent. Figs. 6A and 6B respectively show the skin condition, before and after, the atopic dermatitis being treated by deodorant composition having piperine as anti-irritating agent. Figs. 7A and 7B respectively show the skin condition, before and after, the atopic dermatitis being treated by deodorant composition having the combination of piperine and matrine as anti-irritating agents.

In summary, the present invention provides a low or reduced skin irritating, hexamine comprising composition for use in a deodorant preparation which demonstrates high potency in reducing, inhibiting or suppressing development of sweat relating malodor. The composition shows little to none irritation even at high hexamine concentration of about 40 w/w%. The present invention further demonstrates potent allergy-treating effect to atopic dermatitis as demonstrated by the various experiments on guinea pigs. The beneficial, low skin irritating effect is achieved by the specific selection of naturally occurring, botanically derived compounds comprising one or more flavonoids, alkaloids, iridoids, terpenes, phenols, or a combination thereof, which can be, but are not limited to, one or more of baicalin, celastrol, quercetin, total paeony of glucosides (TPG), curcumin, neohesperidin, piperine, paeoniflorin, hispidulin, glycyrrhizin, matrine and/ore geniposide. The buffer system of the present invention further stabilizes hexamine in acidic sweat to reduce break down and the subsequent release of formaldehyde from hexamine, which are known to be a skin-irritating agent. The buffer system further provides a subsidiary neutralization effect to any odoriferous acids results from the bacterial decomposition of organic matters in sweat, which further assists in reducing or preventing the development of malodor.

The present description illustrates the principles of the present invention. It will thus be appreciated that those skilled in the art will be able to devise various arrangements that, although not explicitly described or shown herein, embody the principles of the invention and are included within its spirit and scope.

Moreover, all statements herein reciting principles, aspects, and embodiments of the invention, as well as specific examples thereof, are intended to encompass both structural, compositional and functional equivalents thereof. Additionally, it is intended that such equivalents include both currently known equivalents as well as equivalents developed in the future, i.e., any elements developed that perform the same function, regardless of structure.

While the invention has been illustrated and described in detail in the drawings and foregoing description, the same is to be considered as illustrative and not restrictive in character, it being understood that only exemplary embodiments have been shown and described and do not limit the scope of the invention in any manner. It can be appreciated that any of the features described herein may be used with any embodiment. The illustrative embodiments are not exclusive of each other or of other embodiments not recited herein. Accordingly, the invention also provides embodiments that comprise combinations of one or more of the illustrative embodiments described above. Modifications and variations of the invention as herein set forth can be made without departing from the spirit and scope thereof, and, therefore, only such limitations should be imposed as are indicated by the appended claims.

In the claims hereof, any element expressed as a means for performing a specified function is intended to encompass any way of performing that function. The invention as defined by such claims resides in the fact that the functionalities provided by the various recited means are combined and brought together in the manner which the claims call for. It is thus regarded that any means that can provide those functionalities are equivalent to those shown herein.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.
It is to be understood that, if any prior art publication is referred to herein, such reference does not constitute an admission that the publication forms a part of the common general knowledge in the art.

## Claims

1. A low-skin irritating deodorant composition, comprising:
an effective amount of an active ingredient comprising hexamine, derivatives thereof, or a combination thereof for reducing, inhibiting or suppressing development of malodour from a body part;
a buffer system for substantially neutralising topical acidity of the body part; and
an anti-irritating agent in an effective amount, wherein the anti-irritating agent comprises one or more botanically derived compounds selected from a group consisting of flavonoids, alkaloids, iridoids, terpenes, phenols, or a combination thereof.

2. The low-skin irritating deodorant composition according to claim 1, wherein the flavonoids of the botanically derived compounds are selected from a group consisting of baicalin, quercetin, neohesperidin, hispidulin or a combination thereof.

3. The low-skin irritating deodorant composition according to claim 1, wherein the alkaloids of the botanically derived compounds are selected from a group consisting of piperine, matrine or a combination thereof.

4. The low-skin irritating deodorant composition according to claim 1, wherein the iridoids of the botanically derived compounds comprise iridoid glycosides; wherein the iridoid glycosides comprise geniposide.

5. The low-skin irritating deodorant composition according to claim 1, wherein the terpenes of the botanically derived compounds are selected from a group consisting of monoterpenes, diterpenes, triterpenes, sesquiterpenes, sesquarterpenes, tetraterpenes or a combination thereof.

6. The low-skin irritating deodorant composition according to claim 5, wherein the monoterpens of the botanically derived compounds comprise total paeony glucosides (TPG) comprising one or more of paeoniflorin, albiflorin, oxypaeoniflorin and benzoylpaeoniflorin; and wherein the triterpenes of the botanically derived compounds comprise celastrol.

7. The low-skin irritating deodorant composition according to claim 1, wherein the phenols of the botanically derived compounds comprise polyphenols; and wherein the polyphenols comprise curcumin.

8. The low-skin irritating deodorant composition according to claim 1, wherein the anti-irritating agent is of about 0.1 w/w% to about 1.0 w/w% in respect of a total weight of the composition.

9. The low-skin irritating deodorant composition according to claim 1, wherein the active ingredient comprising hexamine, derivatives thereof or a combination thereof is of about 1.0 w/w% to about 42.0 w/w% in respect of a total weight of the composition.

10. The low-skin irritating deodorant composition according to claim 1, wherein the buffer system is adapted to maintain pH of the composition in a range of about 7.0 to about 11.0 after the composition is applied to the body part.

11. The low-skin irritating deodorant composition according to claim 1, wherein the composition is provided in a topically administered preparation in one or more of the following forms: a solid, a gel, a cream, a powder, a liquid, a spray and a wipe.

12. A deodorant preparation for use at a body part of a subject, the preparation comprising the composition according to claim 1.

13. A method of preparing a low skin-irritating deodorant composition according to claim 1, comprising:
providing a buffer solution;
dissolving an effective amount of hexamine, derivatives thereof, or a combination thereof in the buffer solution to form a hexamine-comprising solution; and
incorporating an anti-irritating agent in an effective amount into the hexamine-comprising solution, wherein the anti-irritating agent comprises one or more botanically derived compounds selected from a group consisting of flavonoids, alkaloids, iridoids, terpenes, phenols, or a combination thereof.

14. The method of preparing a low skin-irritating deodorant composition according to claim 13, further comprising including a solvent and/or a humectant into the hexamine-comprising solution prior to incorporating the anti-irritating agent into the hexamine-comprising solution.

15. The method of preparing a low skin-irritating deodorant composition according to claim 13, further comprising including one or more excipients into the hexamine-comprising solution after the incorporating of the anti-irritating agent into the hexamine-comprising solution.

## Amended claims

### Amended claims in accordance with Rule 137(2) EPC.

1. A low-skin irritating deodorant composition, comprising:
an active ingredient comprising hexamine of 1.0 w/w% to 42.0 w/w% in respect of a total weight of the composition adapted to reduce, inhibit or suppress development of malodour from a body part;
a buffer system comprising one or more of an inorganic compound and an organic compound adapted to maintain pH of the composition in a range of 7.0 to 11.0; and
an anti-irritating agent in an effective amount, wherein the anti-irritating agent comprises one or more botanically derived compounds selected from a group consisting of: neohesperidin, hispidulin, total paeony glucosides (TPG), glycyrrhizin or a combination thereof.

2. The low-skin irritating deodorant composition according to claim 1, wherein the anti-irritating agent further comprises one or more botanically derived compounds selected from a group consisting of: baicalin, quercetin, piperine, matrine, geniposide, paeoniflorin, celastrol, curcumin and a combination thereof.

3. The low-skin irritating deodorant composition according to claim 1, wherein the anti-irritating agent is of 0.1 w/w% to 1.0 w/w% in respect of a total weight of the composition.

4. The low-skin irritating deodorant composition according to claim 1, wherein the one or more of an inorganic compound and an organic compound of the buffer system are selected from the group consisting of 3-Morpholinopropane-1-sulfonic acid (MOPS), 4-(2-hydroxyethyl)-1-piperazineethanesulfonic acid) (HEPES), 3-(cyclohexylamino)-1-propanesulfonic acid (CAPS), 3-[[1,3-dihydroxy-2-(hydroxymethyl)propan-2-yl]amino]-2-hydroxypropane-1-sulfonic acid (TAPSO), 2-Amino-2-(hydroxymethyl)-1,3-propanediol (Trizma), a phosphate compound, the salts thereof, or a combination thereof.

5. The low-skin irritating deodorant composition according to claim 1, wherein the composition is provided in a topically administered preparation in one or more of the following forms: a solid, a gel, a cream, a powder, a liquid, a spray and a wipe.

6. A deodorant preparation for use at a body part of a subject, the preparation comprising the composition according to claim 1.

7. A method of preparing a low skin-irritating deodorant composition according to claim 1, comprising:
providing a buffer solution adapted to maintain pH of the composition in a range of 7.0 to 11.0;
dissolving hexamine of 1.0 w/w% to 42.0 w/w% in respect of a total weight of the composition in the buffer solution to form a hexamine-comprising solution; and
incorporating an anti-irritating agent in an effective amount into the hexamine-comprising solution, wherein the anti-irritating agent comprises one or more botanically derived compounds selected from a group consisting of neohesperidin, hispidulin, total paeony glucosides (TPG), glycyrrhizin or a combination thereof.

8. The method of preparing a low skin-irritating deodorant composition according to claim 7, wherein the anti-irritating agent further comprises one or more botanically derived compounds selected from a group consisting of baicalin, quercetin, piperine, matrine, geniposide, paeoniflorin, celastrol, curcumin and a combination thereof.

9. The method of preparing a low skin-irritating deodorant composition according to claim 7, further comprising including a solvent and/or a humectant into the hexamine-comprising solution prior to incorporating the anti-irritating agent into the hexamine-comprising solution.

10. The method of preparing a low skin-irritating deodorant composition according to claim 7, further comprising including one or more excipients into the hexamine-comprising solution after the incorporating of the anti-irritating agent into the hexamine-comprising solution.
